# EUROPÄISCHE PATENTSCHRIFT

(11) **EP 3 253 359 B1**
(45) Veröffentlichungstag und Bekanntmachung des Hinweises auf die Patenterteilung: **20.06.2018**
(21) Anmeldenummer: 16700887.9
(22) Anmeldetag: 18.01.2016
(51) Int. Cl.: A61K 8/02, A61K 8/60, A61Q 11/00

(54) **PULVER UND VERFAHREN ZUR REINIGUNG VON ZÄHNEN**
POWDER AND METHOD FOR CLEANING TEETH
POUDRE ET PROCÉDÉ POUR LE NETTOYAGE DES DENTS

(30) Priorität: 03.02.2015 DE 102015201871
(43) Veröffentlichungstag der Anmeldung: 13.12.2017
(73) Patentinhaber: Ferton Holding S.A., 2800 Delémont (CH)
(72) Erfinder: DONNET, Marcel, 01630 Saint Jean de Gonville (FR); SAUVAGEOT-MAXIT, Karine, 01420 Corbonod (FR)
(74) Vertreter: Müller Schupfner & Partner Patent- und Rechtsanwaltspartnerschaft mbB
(86) Internationale Anmeldenummer: PCT/EP2016/050892
(87) Internationale Veröffentlichungsnummer: WO 2016/124381

(56) Entgegenhaltungen:
- EP-A1- 2 228 175
- EP-A1- 2 572 699
- WO-A1-2014/025355
- DE-U1- 20 009 665
- DE-U1- 29 810 580
- US-A1- 2012 121 734
- US-B1- 6 322 774
- DATABASE GNPD [Online] MINTEL; 31. Mai 2000 (2000-05-31), McNeil Consumer Products: "Splenda No Calorie Sweetener", XP002754485, gefunden im Mintel Database accession no. 10066437
- ALDEEB OMAR A A ET AL: "CHAPTER 10: Sucralose", 1. Januar 2013 (2013-01-01), PROFILES OF DRUG SUBSTANCES, EXCIPIENTS AND RELATED METHODOLOGY, ELSEVIER, PAGE(S) 423 - 462, XP009175960, ISBN: 978-0-12-407691-4 Kapitel A und D
- DATABASE GNPD [Online] MINTEL; 28. Februar 2005 (2005-02-28), Melaleuca: "Whitening Tooth Polish", XP002754486, gefunden im Mintel Database accession no. 10208461

## Beschreibung

Die Erfindung betrifft ein Pulver zur Verwendung in einem Pulverstrahlgerät zur Reinigung einer Zahnoberfläche durch Pulverstrahlen, wobei das Pulver zur Reinigung der Zahnoberfläche zusammen mit einem gasförmigen Trägermedium, insbesondere Luft und ggf. einem Fluid, wie Wasser, auf die Zahnoberfläche gestrahlt wird. Die Erfindung betrifft des Weiteren ein Verfahren zur Reinigung von Zähnen unter Verwendung eines Pulverstrahlgeräts, mittels welchem ein Pulver zusammen mit einem gasförmigen Trägermedium, insbesondere Luft und ggf. einem Fluid, wie Wasser, auf eine Zahnoberfläche gestrahlt wird.

Derartige Zahnreinigungspulver bzw. Dentalpulver und entsprechende Verfahren zur Zahnreinigung sind im Stand der Technik bekannt. Hierbei wird das Pulver gemeinsam mit einem gasförmigen Trägermedium, üblicherweise Luft, auf die Zahnoberfläche gestrahlt, wodurch eine besonders effiziente Reinigung der Zahnoberfläche erzielt wird. Zusätzlich oder alternativ zu einem gasförmigen Trägermedium kann grundsätzlich auch ein flüssiges Trägermedium, beispielsweise Wasser, verwendet werden.

Ein Zahnreinigungspulver der genannte Art ist beispielsweise in der DE 200 09 665 U1 beschrieben. Das Pulver enthält ein Basispulver aus Natriumbicarbonat, alternativ Aluminiumoxid oder Dolomit, sowie zusätzliche Wirkstoffe, welche z.B. eine antimikrobielle, desinfizierende, geschmackliche oder zu einer Remineralisierung der Zähne beitragende Wirkung aufweisen.

Ein weiteres Pulver zur Verwendung in einem Pulverstrahlgerät ist in der EP 2 228 175 A1 beschrieben. Dieses Pulver basiert auf Alditolen, insbesondere Mannitol und/oder Erythritol.

Die DE 29 30 836 A1 beschreibt ein Verfahren zur Reinigung von Zähnen, bei welchem die Partikel mittels eines Gasstroms auf die Oberfläche eines zu reinigenden Zahnes gerichtet werden und gleichzeitig ein den Gasstrom umgebender Wasserstrom auf diese Oberfläche gerichtet wird.

In der DE 100 26 718 A1 ist ein geeignetes dentales Abrasivstrahlgerät für ein Dentalpulver, beispielsweise aus Natriumbicarbonat, beschrieben.

XP 002754485 (Mintel; 31. Mai 2000, McNeil Consumer Products: "Splenda No Calories Sweetner") betrifft eine Produktbeschreibung des Süssstoffes "Splenda" des Unternehmens McNeil Consumer Products. In dieser Produktbeschreibung wird das Produkt Splenda, welches aus Maltodextrin und Sucralose besteht, als Null-Kalorien Süssungsmittel beworben, welches zum Kochen und Backen genutzt werden kann.

XP 009175960 (ALDEEB OMAR A. A. et al.: "Chapter 10: Sucralose", 1. Januar 2013, Profiles of drug substances, excipients and related methodlogy, Elsevier, Seiten 423-462, ISBN: 978-0-12-407691-4) betrifft eine wissenschaftliche Arbeit zu Sucralose, in welcher chemische und physikalische Eigenschaften dieser Substanz zusammengefasst präsentiert werden.

XP 002754486 (Mintel; 28. Februar 2005, Melaleuca: "Whitening Tooth Polish") betrifft eine Produktbeschreibung des Poliermittels Melaleuca vom gleichnamigen Unternehmen in Form einer Paste. In der Paste sind viele Inhaltstoffe enthalten, so auch das Alditol Sorbit.

EP 2 572 699 A1 offenbart ein Pulvergemisch mit Calcium-Carbonat-Partikeln sowie ein Verfahren zur Reinigung von Zähnen.

DE 298 10 580 U1 offenbart ein Pulver zur Behandlung von Zahnoberflächen und insbesondere ein für Pulverstrahlinstrumente geeignetes Pulver zur Behandlung von Zahnoberflächen.

WO 2014/025355 A1 offenbart eine Zahnpaste, welche Wasserstoffperoxid und Zitronensäure und Phosphorsäure umfasst. Ferner umfasst die Zahnpaste unter anderem Sucralose und Saccharin.

US 6, 322, 744 B1 offenbart ein Zahnbleichmittel, welches Sucralose zum Maskieren eines Bittergeschmacks enthält.

US 2012/121734 A1 offenbart eine Zusammensetzung, welche Sucralose enthält. Die Zusammensetzung ist unter anderem als ein Mittel zur Maskierung unangenehmen Geschmacks einsetzbar.

Der Erfindung liegt die Aufgabe zu Grunde, ein Pulver zur Zahnreinigung und ein Verfahren zur Zahnreinigung anzugeben, welche eine effiziente und möglichst wenig unangenehme Zahnreinigung ermöglichen. Der Erfindung liegt weiterhin die Aufgabe zu Grunde, ein Zahnreinigungspulver mit einer verbesserten Lagerfähigkeit und einer verbesserten Verteilung im Gasstrom zur Verfügung zu stellen, d.h. ein Pulver, das sich länger lagern lässt und das besser verwirbelt.

Die Aufgabe wird erfindungsgemäß durch ein Pulver (Zahnreinigungspulver, Dentalpulver) mit den Merkmalen des Anspruchs 1, ein Verfahren zur Reinigung von Zähnen mit den Merkmalen des Anspruchs 8 sowie die Verwendung gemäß Anspruch 11 gelöst. Bevorzugte Ausgestaltungen der Erfindung sind Gegenstand der abhängigen Ansprüche sowie der nachfolgenden Beschreibung.

Das erfindungsgemäße Pulver zur Zahnreinigung ist dadurch gekennzeichnet, dass es Sucralose enthält. Das Verfahren ist in entsprechender Weise dadurch gekennzeichnet, dass das verwendete Pulver Sucralose enthält. Das Pulver wird erfindungsgemäß als Mittel zur Pulverstrahlbearbeitung, insbesondere Pulverstrahlreinigung, von Zahnoberflächen, insbesondere Zahnschmelz und/oder Dentin, verwendet.

Das Pulver enthält Sucralose und mindestens 90 Gew.-% Natriumhydrogencarbonat, Erythritol und/oder Glyzin und ist dazu geeignet und vorgesehen, in Pulverform zusammen mit einem gasförmigen Trägermedium, vorzugsweise Luft, auf die Zahnoberfläche (supragingival oder subgingival) gestrahlt zu werden. Insbesondere ist das Pulver zur Verwendung in einem Abrasivstrahlgerät (Pulverstrahlgerät, Strahldruckgerät) vorgesehen und geeignet. Dabei findet eine abrasive bzw. reinigende Behandlung von Zahnoberflächen durch das Auftreffen der Partikel des Pulvers in dem Luftstrahl statt. Ein Pulverstrahlgerät umfasst eine Pulverkammer, in welcher das Pulver mittels eines erzeugten Luftstrahls aufgewirbelt wird. Das entstehende Luft-Pulver-Gemisch wird dann über eine Leitung und eine Austrittsdüse auf die zu reinigende Zahnoberfläche gerichtet. Hierbei werden durch das Auftreffen der Partikel unter Druck auf die Zahnoberfläche Ablagerungen auf der Zahnoberfläche entfernt.

Die Sucralose in dem Zahnreinigungs- bzw. Dentalpulver hat mehrere vorteilhafte Wirkungen. In erster Linie verhindert die Sucralose eine Bindung von Feuchtigkeit in dem Pulver, so dass das Pulver länger lagerfähig bleibt. Darüber hinaus hat sich gezeigt, dass durch die Sucralose, im Vergleich zu beispielsweise Zucker oder Saccharin, eine Verbesserung des Geschmacks des Pulvers erreicht werden kann. Schließlich verbessert Sucralose die Körnigkeit des Pulvers, d.h. es verwirbelt besser in der Pulverkammer und verteilt sich auch besser im Gasstrom. Die Reinigungs- bzw. Abrasivwirkung am Zahn ist gleichmäßiger, es kommt nicht zu einem unerwünschten Pulsieren des Pulver-/Luftstrahls.

Die Verwendung von Sucralose in dem Zahnreinigungspulver ermöglicht daher die Verwendung eines Basispulvers aus abrasiven Partikeln, welches zur Aufnahme von Wasser tendiert (hygroskopisch ist) und/oder einen unangenehmen Geschmack aufweist bzw. klumpt oder zum Klumpen neigt. Insbesondere werden die Eigenschaften eines Zahnreinigungspulvers auf Basis eines wasserlöslichen Basispulvers, beispielsweise Natriumhydrogencarbonat (Natriumbicarbonat), verbessert. Einerseits ist es erwünscht, dass sich das Pulver nach dem Auftreffen auf die Zahnoberfläche im Mund des Patienten auflöst; andererseits ist eine gute Lager fähigkeit angestrebt. Durch die Beimischung von Sucralose zu dem Zahnreinigungspulver können beide Eigenschaften in vorteilhafter Weise miteinander verbunden werden.

Darüber hinaus kann der salzige, zumeist als unangenehm empfundene Geschmack von beispielsweise Natriumbicarbonat (Natriumhydrogencarbonat) durch die Sucralose erheblich besser überdeckt bzw. verdeckt werden als beispielsweise durch Zucker oder andere Süßstoffe wie Saccharin. Das bisher üblicherweise verwendete Saccharin hatte den Nachteil, dass eine Überdosierung zu einem bitteren Geschmack führte, während eine "normale" Dosierung den unangenehmen Geschmack des Natriumbicarbonats nicht angemessen überdeckt. Darüber hinaus haben die meisten Süßstoffe in der Mischung mit Salz einen unangenehmen Geschmack. Sucralose hingegen weist diese Nachteile nicht auf.

Versuche haben gezeigt, dass die Rieselfähigkeit des Basispulvers mit zunehmender Lagerdauer durch die Feuchtigkeitsaufnahme abnimmt. Die Zugabe von gängigen Süßstoffen, wie z.B. Saccharin, fördert die Feuchtigkeitsaufnahme des Pulvers darüber hinaus und ist daher zumindest für diese Pulvereigenschaft kontraproduktiv. Pulver mit einer Beimischung von Sucralose hingegen zeigen diese negativen Eigenschaften nicht. Die Rieselfähigkeit bleibt deutlich länger erhalten. Dies ist insbesondere für die Verwendung in Pulverstrahlgeräten wichtig, da die Verwirbelung in der Pulverkammer nur mit einem ausreichend rieselfähigen Pulver zu zufriedenstellenden Ergebnissen führt.

Durch die Erfindung wird somit ein gut lagerfähiges und geschmacklich angenehmes Dentalpulver zur Verfügung gestellt, das gleichzeitig körniger ist.

Das Pulver enthält ein Basispulver, insbesondere mindestens 90 Gew.-% eines abrasiven Basispulvers zur Zahnreinigung. Das Basispulver enthält abrasiv wirkende Partikel zur Zahnreinigung bzw. besteht aus solchen abrasiven Partikeln. Der Gewichtsanteil des Basispulvers an dem gesamten Pulver beträgt vorzugsweise mindestens 90 Gew.-%, insbesondere mindestens 95 Gew.-%, be sonders bevorzugt mindestens 97 Gew.-%. Das Basispulver enthält insbesondere Natriumhydrogencarbonat (Natriumbicarbonat) und/oder ein Alditol wie beispielsweise Erythritol und/oder Glycin. Auch andere Stoffe, die für dentale Pulverstrahlgeräte als Basispulver zum Einsatz kommen, sind geeignet, wie z.B. Kalziumcarbonat oder Aluminiumtrihydroxid. Vorzugsweise besteht das Basispulver aus mindestens einem Stoff ausgewählt aus der Gruppe von Natriumhydrogencarbonat, einem Alditol wie beispielsweise Erythritol und/oder Glycin.

In einer bevorzugten Ausführungsform der Erfindung enthält das Pulver vorzugsweise mindestens 95 Gew.-%, besonders bevorzugt mindestens 97 Gew.-% Natriumhydrogencarbonat, Erythritol und/oder Glycin. Die genannten Stoffe wirken abrasiv und bilden somit das Mittel zur Zahnreinigung durch ihr Aufstrahlen auf die Zahnoberfläche, d.h. das Basispulver. Dem Basispulver können gegebenenfalls Zusätze beigemischt werden. Als Basispulver können grundsätzlich aber auch andere Stoffe, wie beispielsweise andere Alditole, verwendet werden.

Im Hinblick auf die wasserbindenden und geschmacklichen Eigenschaften hat sich als vorteilhaft herausgestellt, dass das Pulver 0,1 Gew.-% bis 3,0 Gew.-%, vorzugsweise 0,1 Gew.-% bis 1,8 Gew.-%, besonders bevorzugt 0,2 Gew.-% bis 1,5 Gew.-% Sucralose enthält.

Vorzugsweise enthält das Pulver zusätzlich amorphes Silika, vorzugsweise etwa 0,1 Gew.-% bis 5 Gew.-%, insbesondere 0,2 Gew.-% bis 3 Gew.-%, besonders bevorzugt 0,3 Gew.-% bis 2,5 Gew.-%. Die Silika-Partikel verbessern die Rieselfähigkeit des Pulvers und vermindern die Feuchtigkeitsaufnahme.

Des Weiteren ist es bevorzugt, dass das Pulver mindestens ein Aroma enthält. Das Aroma kann grundsätzlich beliebig gewählt werden und beispielsweise Minze oder Menthol enthalten. Das Aroma ist vorzugsweise auf den Geschmack des Basispulvers (z.B. Natriumhydrogencarbonat) und/oder der Sucralose abgestimmt. Das Aroma kann in einer besonders geringen Konzentration vorliegen, da ein ge gebenenfalls unangenehmer Geschmack des Basispulvers, insbesondere von Natriumhydrogencarbonat, durch das Aroma nicht abgedeckt werden braucht. Dies übernimmt erfindungsgemäß die Sucralose in dem Zahnreinigungspulver. Vorzugsweise enthält das Pulver 0,05 Gew.-% bis 1,5 Gew.-% eines Aromas, besonders bevorzugt 0,1 Gew.-% bis 1,2 Gew.-%.

Erfindungsgemäß ist es insbesondere bevorzugt, dass die Sucralose mit einer Konzentration in dem Pulver vorhanden ist, dass der Geschmack des Basispulvers, insbesondere von Natriumhydrogencarbonat, unter die Wahrnehmungsgrenze abgedeckt bzw. reduziert wird.

Für eine schonende und gleichsam effiziente Zahnreinigung ist es bevorzugt, dass die Partikel des Basispulvers eine mittlere Korngröße von 10 µm bis 100 µm, insbesondere 10 µm bis 65 µm aufweisen. Grundsätzlich sind aber auch Korngrößen von bis zu 200 µm denkbar. Die abrasiven Partikel des Basispulvers können insbesondere an das vorgesehene Anwendungsgebiet des Zahnreinigungspulvers angepasst sein. Beispielsweise ist für eine Zahnreinigung der subgingivalen Zahnflächen eine geringere Korngröße, insbesondere etwa 10 µm bis 30 µm bevorzugt. Für eine Zahnreinigung der supragingivalen Zahnflächen ist eine größere Korngröße, insbesondere etwa 40 µm bis 65 µm bevorzugt.

In einer weiteren bevorzugten Ausführungsform des erfindungsgemäßen Zahnreinigungspulvers ist das Basispulver wasserlöslich. Mit anderen Worten sind die Partikel zur Reinigung der Zahnoberfläche (Partikel des Basispulvers bzw. abrasive Partikel) wasserlöslich. Dadurch lösen sich diese im Mund des Patienten auf und können so nach Erfüllung ihrer Aufgabe zur Zahnreinigung auf einfache Weise abtransportiert werden.

Es hat sich bei Versuchen herausgestellt, dass Sucralose die besondere Eigenschaft hat, sich deutlich schneller in Wasser zu lösen, als andere Süßstoffe, wie z.B. Saccharin. Damit kann die Sucralose auch schneller ihre geschmacksverbessernden Eigenschaften entfalten und den salzigen Geschmack des gut löslichen Natriumhydrogencarbonats kompensieren. Durch die gute und schnelle Löslichkeit der Sucralose reichen bereits geringe Mengen aus, um diese Wirkung zu erzielen. Eine besonders bevorzugte Kombination ist daher die Beimischung von Sucralose zu einem Natriumhydrogencarbonat-Pulver. Der salzige Geschmack des Natriumhydrogencarbonat-Pulvers wird mittels der Sucralose besonders schnell und wirksam durch einen angenehmen süßen Geschmack kompensiert. Dabei wird kein bitterer Nebengeschmack erzeugt.

Es ist bevorzugt, dass das Pulver Sucralose in Pulverform (in Form von Pulver) enthält. Das Pulver enthält also abrasive Partikel zur Reinigung der Zahnoberfläche (Basispulver) und Partikel im Wesentlichen aus Sucralose (Sucralose-Teilchen bzw. Sucralose-Partikel). Das Pulver enthält somit mindestens zwei unterschiedliche Partikel oder Partikelarten, nämlich zum einen Reinigungspartikel und zum anderen Sucralose-Partikel (Pulvergemisch). Vorzugsweise sind die Reinigungspartikel und die Sucralose-Partikel jeweils in Wasser löslich und, insbesondere hinsichtlich ihrer Oberfläche und/oder Partikelgröße und Menge, so aufeinander abgestimmt, dass sich im Wesentlichen eine Lösungsgeschwindigkeit in Wasser einstellt, bei der sich die beiden Geschmacksrichtungen kompensieren. Hierunter kann insbesondere auch verstanden werden, dass die Gesamtheit der abrasiven Partikel (Natriumhydrogencarbonat bzw. Natriumhydrogencarbonat-Pulver) und die Gesamtheit der Sucralose bzw. Sucralose-Partikel nach in etwa der gleichen Zeit aufgelöst ist. Dies optimiert den Geschmack für den Patienten, da der Geschmack der sich lösenden Reinigungspartikel durch die sich gleichzeitig bzw. parallel hierzu lösenden Sucralose-Partikel abgedeckt wird.

Als geeignet haben sich Sucralose-Partikel bzw. Sucralose-Teilchen gezeigt mit einer mittleren Größe zwischen 5 und 40 µm und als besonders geeignet mit einer mittleren Teilchengröße zwischen 10 und 25 µm. Aber auch andere Teilchengrößen sind verwendbar.

In einer weiteren bevorzugten Ausführungsform enthält das Pulver abrasive Partikel zur Reinigung der Zahnoberfläche (Basispulver), wobei die Sucralose in den abrasiven Partikeln (Basispulver) enthalten ist. Dies könnte z.B. durch Agglomeration, Beschichtung oder Fällung erreicht werden, mit einer Sprühtrocknung oder im Fließbett. Auch andere bekannte Verfahren oder Methoden zur Kombination der beiden Stoffe sind denkbar.

In einer besonders bevorzugten Ausgestaltung des erfindungsgemäßen Verfahrens zur Zahnreinigung ist vorgesehen, dass das Pulver wasserlösliche abrasive Partikel enthält, dass die wasserlöslichen, abrasiven Partikel insbesondere Natriumhydrogencarbonat enthalten, dass ein Pulver-Luft-Gemisch und ein Flüssigkeitsstrahl separat auf eine Zahnoberfläche gestrahlt werden, dass das Pulver Sucralose enthält und dass die Sucralose gleichzeitig und insbesondere mit der gleichen Lösungsgeschwindigkeit nach Auftreffen auf die Zahnoberfläche gelöst wird. Hierzu wird vorzugsweise eine Düseneinrichtung eines Pulverstrahlgeräts verwendet, welche eine erste Düse für das Pulver-Luft-Gemisch und eine zweite Düse für den Flüssigkeitsstrahl, insbesondere Wasserstrahl, aufweist. Die zweite Düse ist vorzugsweise um die erste Düse herum, insbesondere ringförmig, angeordnet. Das Pulver-Luft-Gemisch trifft zunächst weitestgehend trocken auf die Zahnoberfläche auf. Anschließend wird das Pulver durch den Wasserstrahl abtransportiert und gelöst. Dabei lösen sich das Natriumhydrogencarbonat und die Sucralose etwa gleichzeitig auf.

### Bevorzugte Ausführungsbeispiele:

Die nachfolgenden Beispiele geben bevorzugte Pulver gemäß der Erfindung wider.

### Ausführungsbeispiel 1:

| | |
|---|---|
| Natriumhydrogencarbonat-Pulver (Partikelgröße 40): | 97,2 Gew.-% |
| Amorphes Silika: | 2,5 Gew.% |
| Aroma: | 0,1 Gew.-% |
| Sucralose: | 0,2 Gew.-% |

### Ausführungsbeispiel 2:

| | |
|---|---|
| Natriumhydrogencarbonat-Pulver (Partikelgröße 70 µm): | 95,0 Gew.-% |
| Amorphes Silika: | 2,5 Gew.% |
| Aroma: | 1,0 Gew.-% |
| Sucralose | 1,5 Gew.-% |

### Ausführungsbeispiel 3:

| | |
|---|---|
| Erythritol (Partikelgröße 13 µm) | 94,8 Gew.-% |
| Amorphes Silika: | 2,5 Gew.-% |
| Aroma: | 1,2 Gew.% |
| Sucralose | 1,5 Gew.-% |

### Ausführungsbeispiel 4:

| | |
|---|---|
| Glyzin (Partikelgröße 20 µm) | 95,0Gew. % |
| Amorphes Silika | 2,5 Gew.% |
| Aroma | 1,0 Gew.-% |
| Sucralose | 1,5 Gew.-% |

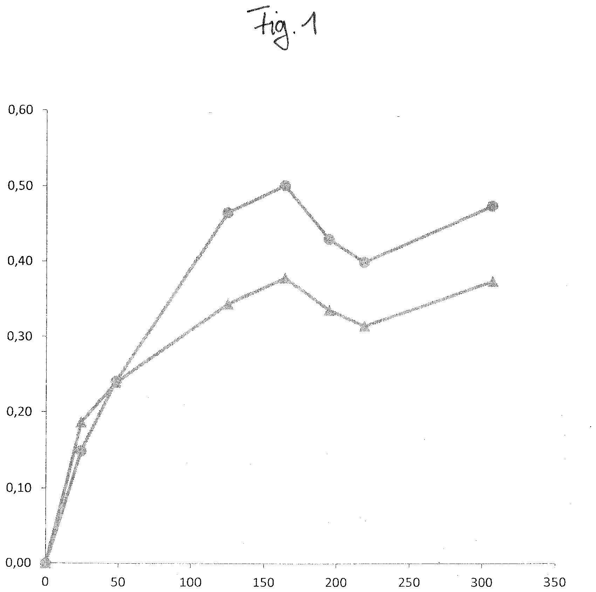

Die Erfindung wird nachfolgend mit Bezug auf die einzige Figur (Fig.1) weiter beschrieben. In der Fig.1 ist eine Messreihe zur Feuchtigkeitsaufnahme eines erfindungsgemäßen Pulvers bzw. einer Pulvermischung (Graph mit dreieckigen Messpunkten) im Vergleich zu einem Referenzpulver (Graph mit runden Messpunkten) dargestellt. Bei dem erfindungsgemäßen Pulver handelt es sich um die Zusammensetzung gemäß Ausführungsbeispiel 1. Bei dem Referenzpulver wurde der Stoff Sucralose durch Saccharin ersetzt. Beide Pulver hatten eine mittlere Korngröße von 40 µm. Die Versuche wurden mit offenen Flaschen bei einer relativen Feuchte von 85% und einer Temperatur von 25° C in einer Klimakammer durchgeführt. Fig. 1 zeigt auf der Abszisse die Zeit in Stunden und auf der Ordinate den Wassergehalt in Gew.-%.

Nach Abschluss der Messungen zeigte die mit Sucralose versetzte Mischung noch ein pulverartiges Verhalten, während die bekannte Pulvermischung mit Saccharine eine pastenartige Konsistenz angenommen hatte. Das Referenzpulver war daher nicht mehr als Strahlmittel zu verwenden.

Die Messreihe zeigt insbesondere, dass eine signifikante Differenz zwischen den beiden Pulvern ab etwa 80 Stunden vorlag. Die maximale Wasseraufnahme des erfindungsgemäßen Pulvers lag bei etwa 0,38 Gew.-%. Hingegen lag die maximale Wasseraufnahme des bekannten Pulvers bei etwa 0,5 Gew.-%.

Es wurde somit gezeigt, dass durch die Beimischung von Sucralose ein Pulver bzw. eine Pulvermischung hergestellt wurde, die aufgrund der Sucralose eine deutlich geringere Wasseraufnahme aufweist.

## Patentansprüche

1. Pulver zur Verwendung in einem Pulverstrahlgerät zur Reinigung einer Zahnoberfläche durch Pulverstrahlen, wobei das Pulver zur Reinigung der Zahnoberfläche zusammen mit einem gasförmigen Trägermedium auf die Zahnoberfläche gestrahlt wird,
**dadurch gekennzeichnet,**
**dass** das Pulver Sucralose und mindestens 90 Gew.-% Natriumhydrogencarbonat, Erythritol und/oder Glyzin enthält.

2. Pulver nach Anspruch 1,
**dadurch gekennzeichnet,**
**dass** das Pulver 0,1 Gew.-% - 3,0 Gew.-% Sucralose enthält.

3. Pulver nach einem der Ansprüche 1 - 2,
**dadurch gekennzeichnet,**
**dass** das Pulver Sucralose in Pulverform enthält, wobei die Sucralose- Teilchen eine mittlere Größe zwischen 5 µm und 40 µm aufweisen.

4. Pulver nach einem der Ansprüche 1 - 3,
**dadurch gekennzeichnet, dass** das Pulver Sucralose in Pulverform enthält, wobei die Sucralose-
Teilchen eine mittlere Größe zwischen 10 µm und 25 µm aufweisen.

5. Pulver nach einem der Ansprüche 1 - 4,
**dadurch gekennzeichnet,**
**dass** das Pulver amorphes Silika enthält.

6. Pulver nach einem der Ansprüche 1-5
**dadurch gekennzeichnet,**
**dass** das Pulver mindestens ein Aroma enthält.

7. Pulver nach einem der Ansprüche 1 - 6,
**dadurch gekennzeichnet,**
**dass** das Pulver ein Basispulver enthält, deren Partikel eine mittlere Korn- große von 10 µm bis 75 µm aufweisen.

8. Pulver nach einem der Ansprüche 1 - 7,
**dadurch gekennzeichnet,**
**dass** das Pulver ein Basispulver enthält, welches wasserlöslich ist.

9. Verfahren zur Reinigung von Zähnen unter Verwendung eines Pulverstrahlgeräts, mit welchem ein Pulver, insbesondere nach einem der Ansprüche 1 - 8 zusammen mit einem gasförmigen Trägermedium auf eine Zahnoberfläche gestrahlt wird,
**dadurch gekennzeichnet**
**dass** das Pulver Sucralose enthält.

10. Verfahren nach Anspruch 9
wobei das Pulver wasserlösliche abrasive Partikel enthält
wobei die wasserlöslichen, abrasiven Partikel insbesondere Natriumhydrogencarbonat enthalten, wobei ein Pulver-Luft-Gemisch und ein Flüssigkeitsstrahl separat auf eine Zahnoberfläche gestrahlt werden,
wobei das Pulver Sucralose enthält und
wobei die Sucralose gleichzeitig und insbesondere mit der gleichen Lösungsgeschwindigkeit nach Auftreffen auf die Zahnoberfläche gelöst wird.

11. Verwendung eines Pulvers nach einem der Ansprüche 1 - 8,
ggf. zusammen mit weiteren feinteiligen Stoffen wie Kieselgel, Bleichmitteln, Analgetika und/oder Bakteriziden als Mittel zur Pulverstrahlbearbeitung, insbesondere Pulverstrahlreinigung von Zahnoberflächen, insbesondere Dentin.

12. Verwendung nach Anspruch 11,
wobei das Mittel zusammen mit Luft und Wasser mittels eines Pulverstrahlgeräts auf die zu bearbeitenden Zahnflächen aufgebracht wird.

## Claims

1. A powder for use in a powder jet device for cleaning a tooth surface by powder spraying, wherein the powder for cleaning the tooth surface is sprayed onto the tooth surface together with a gaseous carrier medium,
**characterized in that**
the powder contains sucralose and at least 90 % by weight of sodium hydrogen carbonate, erythritol and/or glycine.

2. The powder according to Claim 1,
**characterized in that**,
the powder contains 0.1 % - 3.0 % by weight of sucralose.

3. The powder according to one of the Claims 1 - 2,
**characterized in that**
the powder contains sucralose in the form of powder, wherein the sucralose particles have an average size between 5 µm and 40 µm.

4. The powder according to one of the Claims 1 - 3,
**characterized in that**
the powder contains sucralose in the form of powder, wherein the sucralose particles have an average size between 10 µm and 25 µm.

5. The powder according to one of the Claims 1 - 4,
**characterized in that**
the powder contains amorphous silica.

6. The powder according to one of the Claims 1-5,
**characterized in that**
the powder contains at least one flavor.

7. The powder according to one of the Claims 1 - 6,
**characterized in that**
the powder contains a base powder, the particles of which have an average grain size of 10 µm to 75 µm.

8. The powder according to one of the Claims 1 - 7,
**characterized in that**
the powder contains a base powder, which is water-soluble.

9. A process for cleaning teeth by the use of a powder jet device, by means of which a powder, especially according to one of the Claims 1 - 8, is sprayed onto a tooth surface together with a gaseous carrier medium,
**characterized in that**
the powder contains sucralose.

10. The process according to Claim 9,
wherein the powder contains water soluble abrasive particles,
wherein the water soluble abrasive particles especially contain sodium hydrogen carbonate,
wherein a powder air mixture and the liquid jet are separately sprayed onto a tooth surface,
wherein the powder contains sucralose, and
wherein the sucralose, following impingement, is applied simultaneously, and especially with the same dissolution rate, onto the tooth surface.

11. Use of a powder according to one of the Claims 1 - 8,
eventually together with other fine-substances, such as silica, bleaching agents, analgetics and/or antibacterial agents, as agents for powder jet processing, especially powder jet cleaning of tooth surfaces, especially dentine.

12. The use according to Claim 11,
wherein the agent is applied onto the tooth surfaces to be processed together with air and water by way of a powder jet device.

## Revendications

1. Poudre destinée à l'utilisation dans un appareil à jet de poudre pour le nettoyage d'une surface dentaire par des jets de poudre, la poudre étant projetée sur la surface dentaire conjointement avec un milieu porteur gazeux pour nettoyer la surface dentaire,
**caractérisée en ce que**
la poudre contient du sucralose et au moins 90 % en poids d'hydrogénocarbonate de sodium, d'érythritol et/ou de glycine.

2. Poudre selon la revendication 1,
**caractérisée en ce que**
la poudre contient 0,1 % en poids à 3,0 % en poids de sucralose.

3. Poudre selon l'une des revendications 1 ou 2,
**caractérisée en ce que**
la poudre contient du sucralose sous forme de poudre, les particules de sucralose présentant une taille moyenne comprise entre 5 µm et 40 µm.

4. Poudre selon l'une des revendications 1 à 3,
**caractérisée en ce que**
la poudre contient du sucralose sous forme de poudre, les particules de sucralose présentant une taille moyenne comprise entre 10 µm et 25 µm.

5. Poudre selon l'une des revendications 1 à 4,
**caractérisée en ce que**
la poudre contient de la silice amorphe.

6. Poudre selon l'une des revendications 1 à 5,
**caractérisée en ce que**
la poudre contient au moins un arôme.

7. Poudre selon l'une des revendications 1 à 6,
**caractérisée en ce que**
la poudre contient une poudre de base dont les particules présentent une granulométrie moyenne de 10 µm à 75 µm.

8. Poudre selon l'une des revendications 1 à 7,
**caractérisée en ce que**
la poudre contient une poudre de base qui est soluble à l'eau.

9. Procédé pour nettoyer des dents en utilisant un appareil à jet de poudre, par lequel une poudre, en particulier selon l'une des revendications 1 à 8, est projetée conjointement avec un milieu porteur gazeux sur une surface dentaire,
**caractérisé en ce que**
la poudre contient du sucralose.

10. Procédé selon la revendication 9,
dans lequel
la poudre contient des particules abrasives solubles à l'eau,
lesdites particules abrasives solubles à l'eau contenant en particulier de l'hydrogéno-carbonate de sodium,
un mélange de poudre et d'air et un jet de liquide sont projetés séparément sur une surface dentaire,
la poudre contient du sucralose, et
le sucralose est dissout simultanément et en particulier à la même vitesse de dissolution après impact sur la surface dentaire.

11. Utilisation d'une poudre selon l'une des revendications 1 à 8, le cas échéant conjointement avec d'autres substances à particules fines, telles que gel de silice, agents de blanchiment, analgésiques et/ou bactéricides en tant qu'agents pour le traitement au jet de poudre, en particulier pour le nettoyage au jet de poudre de surfaces dentaires, en particulier de la dentine.

12. Utilisation selon la revendication 11,
dans laquelle l'agent est appliqué conjointement avec de l'air et de l'eau au moyen d'un appareil à jet de poudre sur les surfaces dentaires à traiter.
